(19) Europäisches Patentamt
European Patent Office
Office européen des brevets

(11) **EP 3 923 810 B1**

(12) **EUROPEAN PATENT SPECIFICATION**

(45) Date of publication and mention
of the grant of the patent:
**03.06.2026 Bulletin 2026/23**

(21) Application number: **20704024.7**

(22) Date of filing: **11.02.2020**

(51) International Patent Classification (IPC):
*A61B 6/00* (2024.01)　　　*A61B 6/50* (2024.01)
*G16H 50/20* (2018.01)　　　*G16H 50/30* (2018.01)
*G16H 30/40* (2018.01)

(52) Cooperative Patent Classification (CPC):
**A61B 6/503; A61B 6/504; A61B 6/5217;
G16H 30/40; G16H 50/20; G16H 50/30;**
A61B 6/4441

(86) International application number:
**PCT/EP2020/053387**

(87) International publication number:
**WO 2020/165120 (20.08.2020 Gazette 2020/34)**

(54) **PREDICTION OF CORONARY MICROVASCULAR DYSFUNCTION FROM CORONARY COMPUTED TOMOGRAPHY**

VORHERSAGE VON KORONARER MIKROVASKULÄRER DYSFUNKTION VON KORONARER COMPUTERTOMOGRAPHIE

PRÉDICTION DE DYSFONCTIONNEMENT MICROVASCULAIRE CORONAIRE PAR TOMOGRAPHIE CORONARIENNE ASSISTÉE PAR ORDINATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.02.2019 EP 19156638**

(43) Date of publication of application:
**22.12.2021 Bulletin 2021/51**

(73) Proprietor: **Koninklijke Philips N.V.
5656 AE Eindhoven (NL)**

(72) Inventors:
• **FREIMAN, Mordechay Pinchas
5656 AE Eindhoven (NL)**
• **GOSHEN, Liran
5656 AE Eindhoven (NL)**

(74) Representative: **Philips Intellectual Property &
Standards
High Tech Campus 52
5656 AG Eindhoven (NL)**

(56) References cited:
**US-A1- 2015 112 182　　US-A1- 2018 276 817
US-B1- 9 349 178**

**Description**

FIELD OF THE INVENTION

The present invention relates to assessing coronary microvascular dysfunction, and in particular to a decision-support system for providing coronary microvascular dysfunction assessment.

BACKGROUND OF THE INVENTION

Many patients who presented to the emergency department with angina symptoms and diagnosed without obstructive coronary artery disease (CAD) on coronary angiography or cardiac computed tomography angiography (CCTA) are diagnosed later on with coronary microvascular dysfunction (CMD). In the past 20 years, a large number of studies using both invasive and non-invasive techniques for the assessment of coronary physiology have produced a large wealth of data leading to a better understanding of CMD and microvascular ischemia. Specifically, invasive coronary flow reserve (CFR) measurements or positron-emission tomography (PET) imaging are considered as gold standard to diagnose CMD. The CFR can be measured either invasively with intracoronary Doppler flow wire under rest and stress conditions or non-invasively myocardial perfusion imaging (MPI) with either single-photon emission computed tomography data (SPECT) imaging or PET imaging. An example of the non-invasively MPI is described in WO 2017/046082 A1. However, either non-invasive imaging or invasive CFR measurements may impose both substantial financial burden and additional risk to patients.

A document US 2018/276817 A1 relates to methods for assessing the presence of functionally significant stenosis in one or more coronary arteries, further known as a severity of vessel obstruction. The methods can implement a prediction phase that comprises segmenting at least a portion of a contrast enhanced volume image data set into data segments corresponding to wall regions of the target organ, and analysing the data segments to extract features that are indicative of an amount of perfusion experiences by wall regions of the target organ. The methods can obtain a feature-perfusion classification model derived from a training set of perfused organs, classify the data segments based on the features extracted and based on the feature-perfusion classification model, and provide, as an output, a prediction indicative of a severity of vessel obstruction based on the classification of the features.

Another document US 9349178 B1 discloses that in hemodynamic determination in medical imaging, a classifier is trained from synthetic data rather than relying on training data from other patients. A computer model, in silico, may be perturbed in many different ways to generate many different examples. The flow is calculated for each resulting example. A bench model, in vitro, may similarly be altered in many different ways. The flow is measured for each resulting example. The machine-learnt classifier uses features from medical scan data for a particular patient to estimate the blood flow based on mapping of features to flow learned from the synthetic data.

SUMMARY OF THE INVENTION

There may be a need to provide a system for a more effective assessment of potential coronary microvascular dysfunction.

The object of the present invention is solved by the subject-matter of the independent claims, wherein further embodiments are incorporated in the dependent claims. It should be noted that the following described aspects of the invention apply also for the decision-support system for providing coronary microvascular dysfunction assessment.

SUMMARY OF THE DISCLOSURE

A first aspect of the disclosre relates to a decision-support system for providing coronary microvascular dysfunction assessment. The decision-support system comprises an input unit, a cardiac anatomy segmentation unit, a perfusion data extraction unit, a coronary microvascular dysfunction prediction unit, and an output unit. The input unit is configured to receive cardiac computed tomography angiography data of a patient and a corresponding scan protocol. The cardiac anatomy segmentation unit is configured to segment the received cardiac computed tomography angiography data to generate a cardiac segmentation map depicting multiple anatomical segments including myocardium segments. The perfusion data extraction unit is configured to extract perfusion data from the received cardiac computed tomography angiography data. The coronary microvascular dysfunction prediction unit is configured to use a statistical model to determine an indicator for a presence of coronary microvascular dysfunction based on the received cardiac computed tomography angiography data, the corresponding scan protocol, and patient-related data including the generated cardiac segmentation map and the extracted perfusion data. The output unit is configured to output the indicator for a presence of coronary microvascular dysfunction.

The term "unit" as used herein may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality.

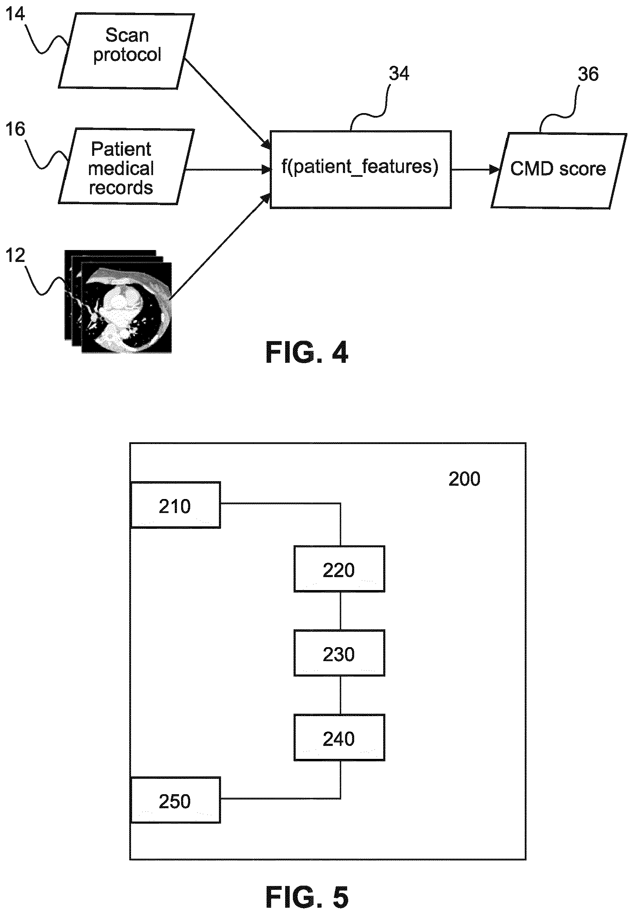

The term "statistical model" as used herein may refer to a set of approaches or models for estimating a function $f$ to map the input to the output. Examples of the approaches may include, but not limited to, partially nonlinear methods, such as generalized additive models, or fully nonlinear methods such as bagging, boosting, and support vector machines with non-linear kernels. The statistical model may be a machine learning model used to find the function e.g. using the supervised-learning methodology. Examples of the machine learning models may include, but not limited to, deep neural networks, regression forests, random forests, and/or support vector machines. This will be explained hereafter and particularly with respect to the example in Fig. 4.

In other words, it is proposed to use a new CCTA result that has the potential to provide reliable prediction of the presence of coronary microvascular dysfunction out from the CCTA data. The CCTA data may be conventional CCTA data or spectral CCTA data, including, but not limited to, photoelectric and scatter data, mono-energetic images, iodine and water maps, virtual non-contrast images, and z-effective maps to characterize CMD. A 3-D cardiac anatomy model is generated from the CCTA data by using a segmentation technique, such as a partial-volume classification approach using voxel histograms, a deformed model-based segmentation, or segmentation with neural networks, to segment the relevant structures from the CCTA data. Perfusion data are also extracted from the CCTA data by calculating perfusion maps describing the overall perfusion of different myocardium segments. A deep-learning based technique may be used to improve the overall accuracy of the CCTA-based perfusion map. The presence of potential CMD may be predicted by combining the 3-D anatomy model, the perfusion data, and a statistical model, such as deep neural networks, regression forests, random forests, support vector machines.

Coronary computed tomography (CT) angiography may be performed to acquire the CCTA data representing a heart or coronary region of the patient. Other CCTA data, such as dual energy or photon counting data may be acquired for the CMD assessment.

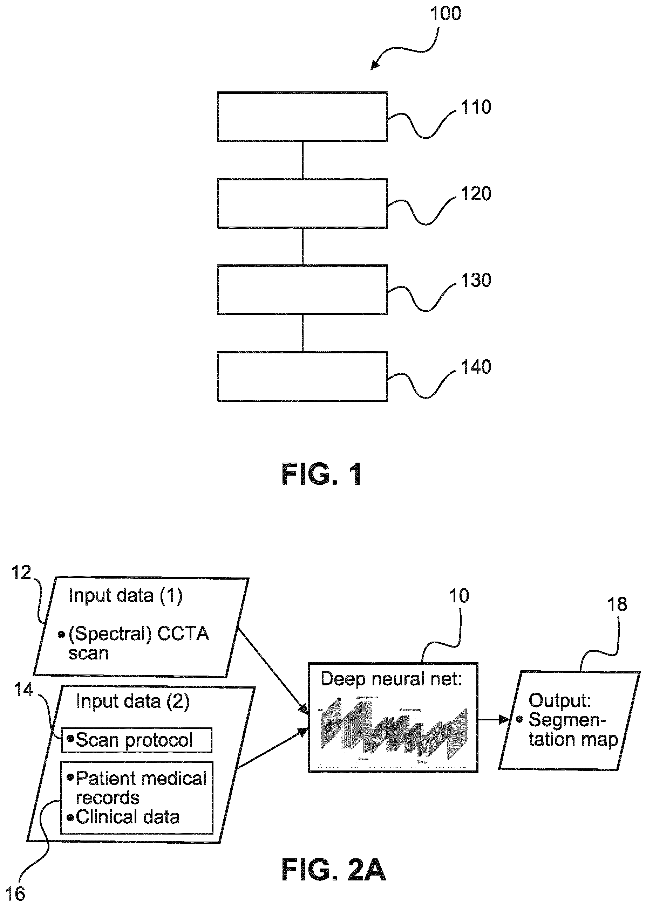

The cardiac anatomy segmentation unit may use various segmentation techniques to generate a 3-D cardiac segmentation model. In an example, the partial volume modeling may be used in automatic coronary lumen segmentation. In another example, the automatic deformed model-based segmentation of the heart in CT images may be used. In a further example, as will be explained hereafter and particularly with respect to the examples of Figs. 2A to 2C, the cardiac anatomy segmentation unit may use a deep learning model to segment the CCTA data. Input to the cardiac anatomy segmentation unit may include, but not limited to, the CCTA scan and the scan protocol. Optionally, the input may further comprise patient medical records and clinical data such as blood pressure and heart rate among others. The cardiac anatomy segmentation unit may determine different anatomical structures within the heart, including the myocardium, the left and right ventricles, the left and right atriums, the ascending and descending aorta, the epicardial fat, the different coronary arteries lumen, wall, plaque, and the surrounding percoronary fat.

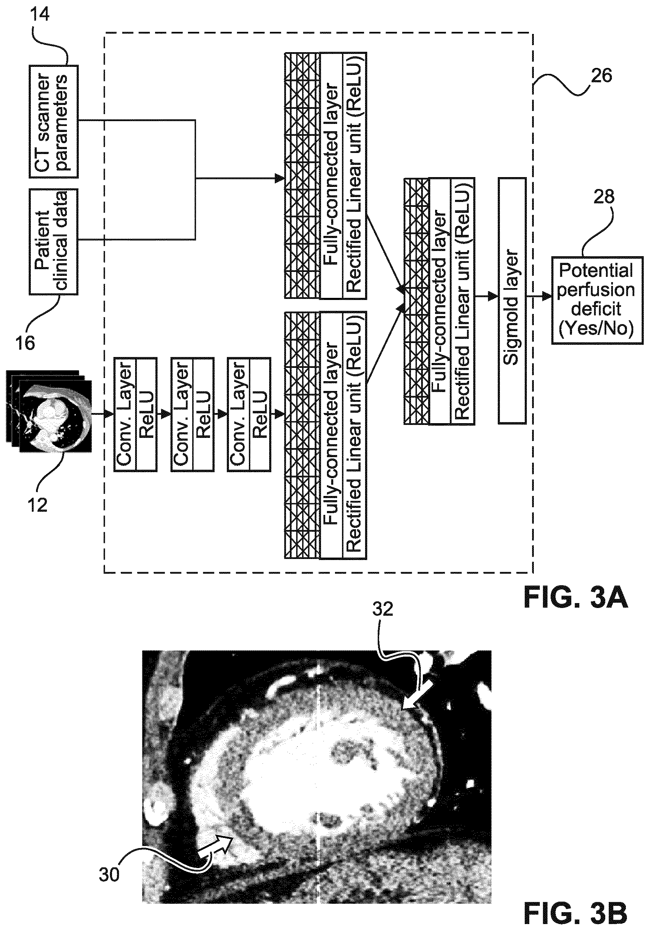

As will be explained hereafter and particularly with respect to the examples of Figs. 3A and 3B, the perfusion data extraction unit may use a deep-learning approach, such as a feedforward neural network or a generative adversarial deep-learning architecture to extract the CCTA-based perfusion data. Input to the perfusion data extraction unit may include, but not limited to, the CCTA scan and the scan protocol. Optionally, the input may further comprise patient medical records and clinical data such as blood pressure and heart rate among others. The extracted myocardial perfusion data may include, but not limited to, a grade representing a probability of a presence of a perfusion deficit and/or a map depicting a probability of a perfusion deficit at each myocardium segment of the cardiac segmentation map.

The coronary microvascular dysfunction prediction unit may use e.g. deep neural networks, regression forests, random forests, support vector machines as the statistical model for the CMD assessment. The input to the coronary microvascular dysfunction prediction unit may include, but not limited to, the CCTA data along with the scan protocol, the 3-D anatomical model, the perfusion analysis results, and any other optional patient data, such as clinical data and patient medical records. The statistical model and the training process will be explained hereafter and particularly with respect to the example of Fig. 4.

This may be beneficial for accelerating the overall patient management and reducing the overall healthcare burden. In particular, a recent study shows that as many as 66% of patients presented to the emergency department with chest pain and diagnosed without obstructive CAD based CCTA data, have been diagnosed later with CMD disease. Currently, only CFR measurements or PET imaging are considered as gold standard to diagnose CMD, which may impose both substantial financial burden and additional risk to patients. To solve the above-mentioned problem, it is proposed to use the CCTA data to enable reliable CMD assessment from CT scans with reduced amount of contrast materials based on a statistical model. In other words, it is proposed to harness the predictive power of a statistical model combined with the CCTA data, the anatomical information extracted from the CCTA data and the perfusion data derived from the CCTA data to predict the presence of CMD directly from the CCTA images rather than requiring additional exams using either non-invasive imaging or invasive CFR measurements. This may enable safe and non-invasive prediction of potential CMD. Additionally, this may reduce the healthcare burden for patients with non-obstructive CMD. Furthermore, as no additional exams are required, the overall patient management may also be accelerated.

According to another example the system is configured to output the indicator for a presence of coronary microvascular dysfunction for non-obstructive coronary vessels, when the CCTA data image contains coronary vessels of a patient without obstructions. In other words, the system and method of this embodiment is capable of detecting and/or indicating coronary microvascular dysfunction despite the fact that no obstructions are present in the CCTA used for this example.

According to an example of the disclosure, the CCTA data comprise conventional cardiac computed tomography angiography data, photon-counting based computed tomography angiography data, and/or spectral cardiac computed tomography angiography data with at least two energy levels.

The CCTA data may include, but not limited to, photoelectric and scatter data, mono-energetic images, iodine and water maps, virtual non-contrast images, and z-effective maps. Spectral CCTA images of the heart reconstructed from CT projection data may be acquired with dual-layer detector system that separate the X-ray flux at the detector into two levels of energy. In addition, spectral CT data may be acquired by a photon-counting scanner. According to an embodiment of the invention, the patient-related data further comprise a patient medical record, and/or clinical data.

The additional patient-related data may provide further information to the CMD assessment and thus improve the accuracy of the prediction.

The term "patient medical records" as used herein may include, without limited to, demographics and patient history.

The term "clinical data" as used herein may refer to any data that may be acquired using invasive or non-invasive techniques. Examples of the non-invasive clinical data can be acquired via a wide range of sensors, comprising medical equipment and devices as well as non-medical grade devices for the measurements of physiological signals. For example, the clinical data may include, but not limited to, blood pressure and heart rate.

According to an example of the disclosure, the perfusion data comprise a grade representing a probability of a presence of a perfusion deficit and/or a map depicting a probability of a perfusion deficit at each myocardium segment of the cardiac segmentation map.

The probability may quantify as a number between 0 and 1, such as 0.25, 0.3, 0.7, etc. The grade or the map may be calculated using standard measures including Hounsfield unit (HU) values or relative enhancement compared to the enhancement in the aorta.

According to an example of the disclosure, the perfusion data extraction unit is configured to use a deep-learning model to extract the perfusion data based on the received cardiac computed tomography angiography data, the corresponding scan protocol, and the patient-related data.

In other words, the perfusion data may be derived directly from the CCTA data without requiring additional exams, such as dynamic perfusion imaging, which may impose additional radiation, contrast material, and imaging artefacts. This will be explained hereafter and particularly with respect to the examples of Figs. 3A and 3B. The term "deep learning" as used herein generally refers to a category of artificial intelligence techniques, which are based on a plurality of information processing layers. Hierarchical structures are employed, either for learning features or representation or for classification/ regression. The deep learning techniques may be categorized into synthesis/generation or recognition/classification. Thus, three main deep learning classes may be considered. Within the first class, discriminative models are developed for delivering high discriminative power either in classification or regression applications. In the second class, generative models are developed for characterizing the higher-order correlation characteristics of the available data for synthesis/analysis. These models may be employed to characterize the mutual statistical properties of the data and the corresponding classes. In the third class, hybrid models may be utilized to perform both discrimination and characterization of the samples.

According to an example of the disclosure, the deep-learning model comprises a feedforward neural network and/or a generative adversarial deep-learning model. An example of the feedforward neural network is illustrated in Fig. 3A and will be explained particularly with respect to this figure.

According to an example of the disclosure, the generative adversarial deep-learning model is configured to generate an enhanced rest-perfusion map so that the perfusion deficit, which is visible on a stress-perfusion map, is visible on the enhanced rest-perfusion map.

The term "generative adversarial deep learning model" as used herein may refer to a class of artificial intelligence algorithms used in unsupervised machine learning, implemented by a system of two neural networks contesting with each other in a zero-sum game framework. The generative network's training objective is to increase the error rate of the discriminative network. In particular, a known dataset serves as the initial training data for the discriminator. Training the discriminator involves presenting it with samples from the dataset, until it reaches some level of accuracy. Typically the generator is seeded with a randomized input that is sampled from a predefined latent space. Thereafter, samples synthesized by the generator are evaluated by the discriminator. Backpropagation is applied in both networks so that the generator produces better images, while the discriminator becomes more skilled at flagging synthetic images. The generator is typically a deconvolutional neural network, and the discriminator is a convolutional neural network.

According to an example of the disclosure, the generative adversarial deep-learning model is configured to be trained using a process for simultaneously training a generator for enhancing a rest-perfusion map and a discriminator for distinguishing between a real stress-perfusion image and an enhanced rest-perfusion image.

According to an example of the disclosure, the deep-learning model is configured to be trained using at least one reference for a perfusion deficit. The at least one reference for the perfusion deficit comprises a cardiac magnetic resonance perfusion measurement, stress perfusion data, single photon emission computed tomography data, and/or positron-emission tomography data.

According to an example of the disclosure, the indicator for a presence of coronary microvascular dysfunction comprises a coronary microvascular dysfunction score. According to an example of the disclosure, the statistical model is a machine-learning model configured to use a function that maps the received cardiac computed tomography angiography data, the corresponding scan protocol, and patient-related data to the indicator for a presence of coronary microvascular dysfunction.

In other words, the function implicitly describes the statistical relation between the input features including, but not limited to, the received CCTA data, the corresponding scan protocol, and patient-related data, and the output CCTA-based CMD score. Such a function may allow the prediction of CMD directly from the CCTA data without the need for additional exams, such as invasive CFR measurements or PET imaging. This may reduce substantial financial burden and additional risk to patients. In addition, this may also accelerate overall patient management.

According to an example of the disclosure, the machine-learning model comprises at least one of the following: a deep neural network, a regression forest, a random forest, and a support vector machine.

These examples of the machine learning models may use the supervised learning methodology to find a function that maps the input features, such as the CCTA data, to the output CMD score.

According to an example of the disclosure, the machine-learning model is configured to use a supervised-learning methodology to create the function that maps an input to an output based on example input-output pairs.

According to an example of the disclosure, the decision-support system further comprises a display configured to display the indicator for a presence of coronary microvascular dysfunction.

According to an example of the disclosure the training process may use a mix structure regularization, wherein the mix structure regularization is a regularization technique for a training phase of a network, which improves the robustness and accuracy of the network. When using mix structure regularization the original input image is corrupted by additional random structure, sampled randomly from the training data (rather than corrupting the image with random noise). In the mix structure regularization meta-parameters are used for controlling the amount of the random structure that is added to the input.

Another aspect of the disclosure relates to a method for providing coronary microvascular dysfunction assessment. The method comprises providing cardiac computed tomography angiography data of a patient and a corresponding scan protocol, segmenting the received cardiac computed tomography angiography data to generate a cardiac segmentation map depicting multiple anatomical segments including myocardium segments, extracting perfusion data from the received cardiac computed tomography angiography data, and using a statistical model to determine an indicator for a presence of coronary microvascular dysfunction based on the received cardiac computed tomography angiography data, the corresponding scan protocol, and patient-related data including the generated cardiac segmentation map and the extracted perfusion data.

In one example, the perfusion data may be extracted from the received CCTA data using a deep-learning model.

In one example, the deep-learning model may comprise a feedforward neural network and/or a generative adversarial deep-learning model.

In one example, the method may further comprise the step of generating an enhanced rest-perfusion map, using the generative adversarial deep-learning model, so that the perfusion deficit, which is visible on a stress-perfusion map, is visible on the enhanced rest-perfusion map.

In one example, the method may further comprise the step of training the generative adversarial deep-learning model using a process for simultaneously training a generator for enhancing a rest-perfusion map and a discriminator for distinguishing between a real stress-perfusion image and an enhanced rest-perfusion image.

In one example, the deep-learning model may be trained using at least one reference for a perfusion deficit. The at least one reference for the perfusion deficit may comprise a cardiac magnetic resonance perfusion measurement, stress perfusion data, single photon emission computed tomography data, and/or positron-emission tomography data. In one example, the statistical model may be a machine-learning model configured to use a function that maps the received coronary computed tomography angiography data, the corresponding scan protocol, and patient-related data to the indicator for a presence of coronary microvascular dysfunction.

In one example, the machine-learning model may comprise at least one of the following: a deep neural network, a regression forest, a random forest, and a support vector machine.

In one example, the method may comprise the step of using a supervised-learning methodology to create the function that maps an input to an output based on example input-output pairs.

In one example, the method may further comprise the step of displaying the indicator for a presence of coronary microvascular dysfunction.

According to another aspect of the disclosure, a computer program element is provided for controlling an apparatus according to one of the embodiments described above and in the following, which, when being executed by a processing unit, is adapted to perform the method.

According to another aspect of the disclosure, a computer readable medium is provided having stored the program element.

According to another aspect of the disclosure, a method is provided for predicting CMD based only on CCTA data that is more reliable and with a reduced amount of contrast materials. The method comprises the following steps:

A) The first step is to provide CCTA data, such as conventional CCTA data or spectral CCTA data. Spectral CCTA data may be preferable, because spectral CT scanner have demonstrated their potential in providing detailed image of the coronary arteries with up to 60% reduction in the amount of contrast material administrated by using the polychromatic information of the X-ray to produce virtually low kVp mono-energetic images. Additionally, spectral CT can also reduce beam-hardening artifacts among others and has the potential to quantify blood perfusion more accurately.

B) The second step is to determine anatomical structures, i.e. segmenting, using an anatomy model based on deep learning. The input comprises acquired CCTA data (step A), the scan protocol, patient medical records and clinical data. Training of the anatomy model includes initializing network parameters randomly and then adjusting them to produce a label map representing the different anatomical structures within the heart as close as possible to the actual segmentation map produced by a human expert according to a predefined loss function.

C) The third step is to determine CCTA based myocardial perfusion data based on deep learning. The input comprises acquired CCTA data (step A), scan protocols, scan parameters and any additional patient data. This step calculates a grade or map representative for the probability of presence of perfusion deficit using standard measures. Additionally, a deep-learning approach predicts perfusion deficit from the CCTA data.

D) The last step is to predict CMD based on deep learning. The input is acquired CCTA data (step A), scan protocols, scan parameters, the anatomical structures (step B), myocardial perfusion data (step C) and any additional patient data. A statistical model predicts the potential presence of CMD from the given data. The model comprises a function that maps patient features onto a CMD score. It therefore describes the statistical relation between the input features and the output CCTA-based CMD score.

The above method may enable the prediction of CMD in patients, who underwent CCTA (and no obstructive CAD was found), directly from CCTA data rather than requiring additional exam either invasive or non-invasive. The invention utilizes either conventional or spectral CT data along with deep-learning based modules, to reconstruct automatically a 3-D cardiac anatomy model, to derive perfusion-related maps and metrics from the CCTA scan, and to predict the potential presence of CMD based on the combination of the CCTA, cardiac anatomy model and perfusion information data. The ability to predict CMD directly from CCTA for patients (without obstructive CAD diagnosis) has the potential to reduce the need for additional exams, to improve patient management and to reduce overall healthcare burden for this patient group.

These and other aspects of the present disclosure will become apparent from and be elucidated with reference to the examples described hereinafter.

BRIEF DESCRIPTION OF THE DRAWINGS

Examples of the disclosure will be described in the following with reference to the following drawings:

Fig. 1 shows a flow diagram of a method for providing coronary microvascular dysfunction assessment according to an example of the present disclosure.
Fig. 2A shows a deep learning model for segmenting the cardiac computed tomography angiography data according to an example of the present disclosure.
Fig. 2B shows a training procedure for the deep learning model according to an example of the present disclosure.
Fig. 2C shows a network structure of the deep learning model according to an example of the present disclosure.
Fig. 3A shows a deep learning model for predicting perfusion deficit according to an example of the present disclosure.
Fig. 3B shows a photo of a cardiac computed tomography angiography scan according to an example of the present disclosure.
Fig. 4 shows a statistical model for predicting the coronary microvascular dysfunction according to an example of the present disclosure.
Fig. 5 schematically shows a decision-support system for providing coronary microvascular dysfunction assessment.

The figures are schematic and not at scale and are only used for graphical purposes.

DETAILED DESCRIPTION OF EMBODIMENTS

CCTA is a rapidly evolving non-invasive technique to rule out CAD due to its high negative predictive value. CCTA is commonly used to characterize the anatomical appearance of existing coronary lesions. However, a large portion of patients diagnosed with non-obstructive CAD based on CCTA still suffer from recurrent chest pain and other ischemia-related symptoms which may indicate CMD. CMD is typically defined as impaired vasodilation of arterioles, leading to an inadequate increase in blood flow from rest state to stress state. Commonly, the coronary flow reserve (CFR) determined by the maximum hyperemic blood flow divided by the baseline coronary flow is used for quantitative assessment of CMD. The CFR can be measured either invasively with intracoronary Doppler flow wire under rest and stress conditions or non-invasively myocardial perfusion imaging (MPI) with either SPECT imaging, PET imaging or Cardiac MR.

Despite the established use of SPECT, PET and Cardiac MR in assessing CFR, none of these imaging technologies is currently able to provide a combined anatomical assessment of the coronary arteries and functional evaluation of the downstream myocardial territory in a single modality. Therefore, these modalities cannot serve as a 'one-stop shop' modality for assessing both CAD and CMD in the same modality.

On the other hand, computed tomography has the potential to provide both anatomical depiction of the major coronaries through CCTA exam and assessing CMD data through either dynamic imaging or rest/stress imaging. Therefore, serving as a 'one-stop shop' for assessing patients with angina symptoms, thus, reducing overall healthcare burden and costs for both patients and the health system. However, the additional radiation, contrast material, and presence of imaging artifacts associated with dynamic perfusion imaging has traditionally reduced its utilization in assessing CMD.

In order to improve the effectiveness of CMD assessment, the following disclosure describes the present invention according to several embodiments directed at methods, systems, and apparatus related to the personalized assessment of the presence of CMD using CCTA data and a statistical model. More specifically, the techniques described herein process the CCTA data to extract the relevant visual information, use the statistical model connecting patient information, including visual features in the CCTA data and other patient-related data and the presence of CMD, and predict the presence of CMD from CCTA data. This may enable safe and non-invasive prediction of potential CMD and healthcare burden reduction for patients with non-obstructive CMD. As no additional exams are required, the overall patient management may also be accelerated.

Fig. 1 illustrates a flow diagram of a method 100 for providing CMD assessment according to some embodiments of the present disclosure. In step 110, as illustrated in Fig. 2A, CCTA data 12 of a patient and a corresponding scan protocol 14 are provided. The CCTA data 12 may include at least either conventional CCTA data or spectral CCTA data, including but not limited to photoelectric and scatter data, mono-energetic images, iodine and water maps, virtual non-contrast images, and z-effective maps. The spectral CCTA data may comprise at least two energy levels that allow spectral analysis. The CCTA images of the heart reconstructed from CT projection data may be acquired with dual-layer detector system that separate the X-ray flux at the detector into two levels of energy. In addition, spectral CT data may be acquired using a photon-counting scanner.

In step 120, the received CCTA data are segmented to generate a cardiac segmentation map depicting multiple anatomical segments including myocardium segments. The cardiac segmentation map may depict multiple anatomical segments within the heart, including the myocardium, the left and right ventricles, the left and right atriums, the ascending and descending aorta, the epicardial fat, the different coronary arteries lumen, wall, plaque, and the surrounding peri coronary fat. Examples of segmentation methods may include, but not limited to, partial-volume classification approaches using voxel histograms, deformed model-based segmentation, and segmentation with neural networks.

One possible segmentation method that may be employed in step 120 is a deep neural network 10 as illustrated in Figs. 2A to 2C. More specifically, Fig. 2A illustrates an example of the deep neural network 10. The input data of the deep neural network 10 may comprise the CCTA data 12, the scan protocol 14 and further optional patient-related data 16, such as patient medical records and clinical data. The output data is a cardiac segmentation map 18 depicting multiple anatomical segments including myocardium segments.

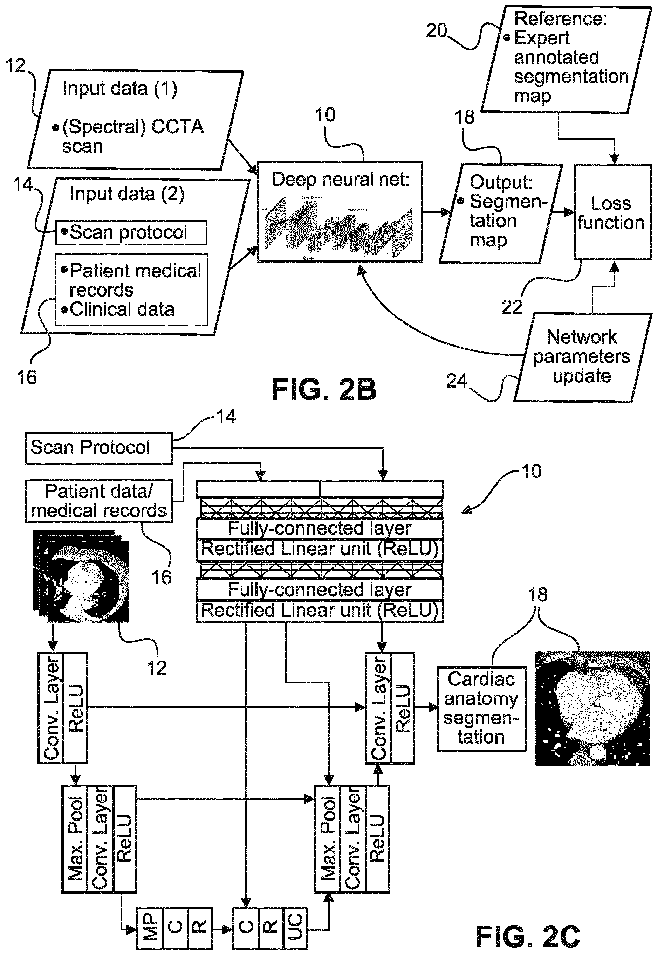

Fig. 2B illustrates an example of the training procedure for the deep neural network 10. To train the deep neural network 10, a reference may be provided containing one or more expert annotated segmentation maps 20. The training procedure may start with initializing the network parameters 24 randomly, then adjust them to produce label map representing the different anatomical structures within the heart as closer as possible to the actual segmentation map produced by a human expert, i.e. the expert annotated segmentation map 20, according to a predefined loss-function 22.

Fig. 2C illustrates one optional detailed network architecture of the deep neural network 10. In this architecture, a U-NET combined with a full-connected network is trained simultaneously to generate the cardiac segmentation map 18. The following generalized Dice loss function may be used for training:

$$DL = 1 - 2\frac{\sum_{l=1}^{L} w_l \sum_n r_{ln}p_{ln}}{\sum_{l=1}^{L} w_l \sum_n r_{ln} + p_{ln}}$$

where $r_{ln}$ is the reference segmentation of pixel $n$, indicates whether pixel $n$ belongs to label $l$, $p_{ln}$ is the network prediction of pixel $n$ to belong to label $l$, and $w_l$ is the relative weight of label $l$ in the data.

Returning to Fig. 1, in step 130, perfusion data are extracted from the received CCTA data. The extracted perfusion data may include, but not limited to, a grade that represents the probability of the presence of perfusion deficit or a map depicting the probability of perfusion deficit at each myocardium segment. The grade or the map can be calculated using standard measures including HU values or relative enhancement compared to the enhancement in the aorta.

Machine learning models may be used in step 130 to extract the perfusion data. In some embodiments, the machine learning models utilized herein may employ deep learning methods, such as a feedforward neural network or a generative adversarial deep-learning architecture.

Fig. 3A illustrates one optional architecture of a feedforward neural network 26 that can be used to predict perfusion deficit 28 from the CCTA data. These models are called feedforward because information flows through the function being evaluated from the input, including but not limited to, the CCTA data 12, the scan protocol 14 and any other optional patient-related data 16, such as patient medical records and clinical data, through the intermediate computations used to define a function, and finally to the output, i.e. the perfusion data 28, including, but not limited to, the grade or the map representing the probability of the presence of perfusion deficit. There are no feedback connections in which outputs of the model are fed back into itself.

The training procedure may utilize any reference for perfusion deficit, including, but not limited to, stress perfusion data, cardiac magnetic resonance perfusion measurements, SPECT data and/or PET data. The following binary cross-entropy loss function can be used for training:

$$Loss_{perfusion} = -ylog(p) + (1 - y)log(1 - p)$$

where $y$ is the reference indicating presence of perfusion deficit, and $p$ is the output probability from the network.

Another option is to use a generative adversarial deep-learning architecture (not shown) to generate enhanced rest-perfusion maps from the CCTA data so that perfusion deficits that are visible on stress-perfusion maps will be visible clearly on the rest-perfusion maps. The training procedure in this case will train simultaneously a generator, which enhance the rest-perfusion map, and a discriminator, which distinguish between real stress-perfusion images and enhanced rest perfusion images.

Fig. 3B illustrates a representative short-axis view of a CCTA scan of a patient with known functionally obstructive CAD at the left anterior descending artery with invasive fractional flow reserve (FFR) less than 0.8, with perfusion deficit already visible in the CCTA data, indicated with an arrow 30. The arrow 32 indicates a healthy region with sufficient blood supply. Similar perfusion features may be used in the CCTA-based machine-learning prediction of CMD.

Returning to Fig. 1, in step 140, a statistical model is used to determine an indicator for a presence of CMD based on the received CCTA data 12, the corresponding scan protocol 14, and patient-related data including the generated cardiac segmentation map 18 and the extracted perfusion data 28. The indicator may be a CMD score. Such a statistical model may be described as a function 34 as illustrated in Fig. 4 that maps the *patient_features* onto CMD score 36:

$$f(patient\_features) \rightarrow CMD\ score$$

where the *patient_features* may include, but not limited to, the CCTA data along with the scan protocol and parameters described in step 110, the 3-D anatomical model described in step 120, the perfusion data described in step 130, and any additional patient data, such as clinical data and patient medical records. The function *f(patient_features)* may be used to estimate the CCTA-based CMD score by determining the *patient_features* as described in steps 110, 120, 130 and applying the function *f(patient_features)* on these *patient_features.* In other words, the function *f(patient_features)* implicitly describes the statistical relation between the input features and the output CCTA-based CMD score. Various possible machine learning models may be employed herein to find the function *f(patient_features)* using e.g. a supervised learning methodology. Examples of the machine learning models to describe and find the function *f(patient_features)* may include, but not limited to, deep neural networks, regression forests, random forests, and/or support vector machines.

To be able to use the statistical model, the model may be trained *a priori* offline. For example, multiple pairs of inputs, namely *patient_features*, and outputs, namely the CMD score, known as training data may be used to find the function *f(patient_features)* using some optimization criteria. As an example, the training procedure may be generally described as:

$$\hat{f} = \arg\min_{f} Error(f(patient\_featuers), CMD_{GT})$$

where *f* is the function mapping the input features onto the CCTA-based CMD score, $CMD_{GT}$ are the expected output values of the function *f*, and *Error* is the machine-learning model that is used. In some models a regularization term is included explicitly in *Error*, and in others regularization to avoid over-fitting to the training data can be achieved using standard techniques, such as cross-validation, or data augmentation. Optimization can be performed using standard techniques including but not limited to the stochastic gradient decent algorithm, among others.

Fig. 5 shows a system 200 for providing CMD assessment according to some embodiments of the present disclosure. The system 200 comprises an input unit 210, a cardiac anatomy segmentation unit 220, a perfusion data extraction unit 230, a coronary microvascular dysfunction prediction unit 240 and an output unit 250.

The input unit 210 is configured to receive CCTA data of a patient and a corresponding scan protocol. The CCTA data may comprise conventional CCTA data, photon-counting based computed tomography angiography data and/or spectral CCTA data with at least two energy levels.

The cardiac anatomy segmentation unit 220 is configured to segment the received CCTA data to generate a cardiac segmentation map depicting different anatomical segments including myocardium segments. One possible segmentation method, embodied as a deep learning based cardiac anatomy modelling, is explained with respect to the exemplary embodiment of Figs. 2A to 2C.

The perfusion data extraction unit 230 is configured to extract perfusion data from the received CCTA data. The perfusion data may comprise a grade representing a probability of a presence of a perfusion deficit and/or a map depicting a probability of a perfusion deficit at each myocardium segment of the cardiac segmentation map. In an embodiment of the system 200 of Fig. 5, the perfusion data extraction unit 230 is configured to use a deep-learning model to extract the perfusion data based on the received CCTA data, the corresponding scan protocol, and the patient-related data. An example of the deep-learning model, embodied as a feedforward neural network, is explained particularly with respect to the exemplary embodiment of Figs. 3A and 3B. Another example of the deep-learning model is a generative adversarial deep-learning model as explained with respect to step 130. The generative adversarial deep-learning model may be configured to generate an enhanced rest-perfusion map so that the perfusion deficit, which is visible on a stress-perfusion map, is visible on the enhanced rest-perfusion map. The generative adversarial deep-learning model may be configured to be trained using a process for simultaneously training a generator for enhancing a rest-perfusion map and a discriminator for distinguishing between a real stress-perfusion image and an enhanced rest-perfusion image. The deep-learning model, e.g. the feedforward neural network or the generative adversarial deep-learning model, may be configured to be trained using at least one reference for a perfusion deficit. The at least one reference for the perfusion deficit comprises a cardiac magnetic resonance perfusion measurement, stress perfusion data, single photon emission computed tomography data, and/or positron-emission tomography data.

The coronary microvascular dysfunction prediction unit 240 is configured to use a statistical model to determine an indicator for a presence of CMD based on the received CCTA data, the corresponding scan protocol, and patient-related data including the generated cardiac segmentation map and the extracted perfusion data. As an example, the indicator for a presence of CMD may comprise a CMD score. Optionally, the statistical model may be a machine-learning model configured to use a function that maps the received CCTA data, the corresponding scan protocol, and patient-related data to the indicator for a presence of CMD. An example of the function is illustrated in Fig. 4. The machine-learning model comprises at least one of the following: a deep neural network, a regression forest, a random forest, and a support vector machine. The machine-learning model is configured to use a supervised-learning methodology to create the function that maps an input to an output based on example input-output pairs.

The output unit 250 is configured to output the indicator for a presence of CMD. As an option, the decision-support system 200 may further comprise a display (not shown) configured to display the indicator for a presence of CMD.

In another example of the present disclosure, a computer program or a computer program element is provided that is characterized by being adapted to execute the method steps of the method according to one of the preceding examples, on an appropriate system.

The computer program element might therefore be stored on a computer unit, which might also be part of an example of the present disclosure. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the disclosure.

Further on, the computer program element might be able to provide all necessary steps to fulfil the procedure of an example of the method as described above.

According to a further example of the present disclosure, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further example of the present disclosure, a medium for making a computer program element available for downloading is provided, which computer program element is arranged to perform a method according to one of the previously described examples of the disclosure.

It has to be noted that examples of the disclosure are described with reference to different subject matters. In particular, some examples are described with reference to method type claims whereas other examples are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A decision-support system (200) for providing coronary microvascular dysfunction assessment, comprising:

   - an input unit (210);
   - a cardiac anatomy segmentation unit (220);
   - a perfusion data extraction unit (230);
   - a coronary microvascular dysfunction prediction unit (240); and
   - an output unit (250);
   
   wherein the input unit is configured to receive coronary computed tomography angiography data (12) of a patient and a corresponding scan protocol (14);
   wherein the cardiac anatomy segmentation unit is configured to segment the received coronary computed tomography angiography data to generate a cardiac segmentation map (18) depicting multiple anatomical segments including myocardium segments;
   wherein the perfusion data extraction unit is configured to extract perfusion data (28) from the received coronary computed tomography angiography data;
   wherein the coronary microvascular dysfunction prediction unit is configured to input the received coronary computed tomography angiography data, the corresponding scan protocol, and patient-related data including the generated cardiac segmentation map and the extracted perfusion data into a statistical model (34) to determine an indicator (36) for a presence of coronary microvascular dysfunction;
   wherein the statistical model is a machine-learning model configured to use a function that maps the received coronary computed tomography angiography data, the corresponding scan protocol, and patient-related data to the indicator for a presence of coronary microvascular dysfunction, wherein the machine-learning model is configured to use a supervised-learning methodology to create the function that maps an input to an output based on example input-output pairs, the example input-output pairs including examples of the coronary computed tomography angiography data, the corresponding scan protocol, and patient-related data including the generated cardiac segmentation map and the extracted perfusion data, as inputs, and examples of the indicator (36), as outputs; and
   wherein the output unit is configured to output the indicator for a presence of coronary microvascular dysfunction.

2. The decision-support system according to claim 1,
   wherein the system is configured to output the indicator for a presence of coronary microvascular dysfunction for non-obstructive coronary vessels, when the CCTA data image contains coronary vessels of a patient without obstructions.

3. The decision-support system according to claim 1 or 2,
   wherein the coronary computed tomography angiography data comprise:

   - conventional coronary computed tomography angiography data;
   - photon-counting based computed tomography angiography data; and/or
   - spectral coronary computed tomography angiography data with at least two energy levels.

4. The decision-support system according to claim 1 to 3,
   wherein the patient-related data further comprise:

   - a patient medical record; and/or
   - clinical data.

5. The decision-support system according to any of the preceding claims,
   wherein the perfusion data comprise:

   - a grade representing a probability of a presence of a perfusion deficit; and/or
   - a map depicting a probability of a perfusion deficit at each myocardium segment of the cardiac segmentation map.

6. The decision-support system according to any of the preceding claims,
   wherein the perfusion data extraction unit is configured to use a deep-learning model to extract the perfusion data based on the received coronary computed tomography angiography data, the corresponding scan protocol, and the patient-related data.

7. The decision-support system according to claim 6,
   wherein the deep-learning model comprises:

   - a feedforward neural network; and/or
   - a generative adversarial deep-learning model.

8. The decision-support system according to claim 7,
   wherein the generative adversarial deep-learning model is configured to generate an enhanced rest-perfusion map so that the perfusion deficit, which is visible on a stress-perfusion map, is visible on the enhanced rest-perfusion map.

9. The decision-support system according to claim 7 or 8,
   wherein the generative adversarial deep-learning model is configured to be trained using a process for simultaneously training a generator for enhancing a rest-perfusion map and a discriminator for distinguishing between a real stress-perfusion image and an enhanced rest-perfusion image.

10. The decision-support system according to any of claims 6 to 8,

    wherein the deep-learning model is configured to be trained using at least one reference for a perfusion deficit; and
    wherein the at least one reference for the perfusion deficit comprises:

    - a cardiac magnetic resonance perfusion measurement;
    - stress perfusion data;
    - single photon emission computed tomography data; and/or
    - positron-emission tomography data.

11. The decision-support system according to claim 10,
    wherein the machine-learning model comprises at least one of the following:

    - a deep neural network;
    - a regression forest;
    - a random forest; and
    - a support vector machine.

12. The decision-support system according to any of the preceding claims, further comprising:

- a display configured to display the indicator for a presence of coronary microvascular dysfunction.

13. A computer program element for controlling the decision-support system according to claim 1, which when executed by a processing unit, is adapted to perform a method (100) for providing coronary microvascular dysfunction assessment, comprising the following steps:

- providing (110) coronary computed tomography angiography data of a patient and a corresponding scan protocol;
- segmenting (120) the received coronary computed tomography angiography data to generate a cardiac segmentation map depicting multiple anatomical segments including myocardium segments;
- extracting (130) perfusion data from the received coronary computed tomography angiography data; and
- inputting (140) the received coronary computed tomography angiography data, the corresponding scan protocol, and patient-related data including the generated cardiac segmentation map and the extracted perfusion data into a statistical model to determine an indicator for a presence of coronary microvascular dysfunction; and

wherein the statistical model is a machine-learning model configured to use a function that maps the received coronary computed tomography angiography data, the corresponding scan protocol, and patient-related data to the indicator for a presence of coronary microvascular dysfunction, wherein the machine-learning model is configured to use a supervised-learning methodology to create the function that maps an input to an output based on example input-output pairs, the example input-output pairs including examples of the coronary computed tomography angiography data, the corresponding scan protocol, and patient-related data including the generated cardiac segmentation map and the extracted perfusion data, as inputs, and examples of the indicator (36), as outputs.

## Patentansprüche

1. Entscheidungsunterstützungssystem (200) zum Bereitstellen einer Beurteilung koronarer mikrovaskulärer Dysfunktion, umfassend:

- eine Eingabeeinheit (210);
- eine Herzanatomie-Segmentierungseinheit (220);
- eine Perfusionsdaten-Extraktionseinheit (230);
- eine Vorhersageeinheit für koronare mikrovaskuläre Dysfunktion (240); und
- eine Ausgabeeinheit (250);

wobei die Eingabeeinheit dazu konfiguriert ist, koronare Computertomographie-Angiographiedaten (12) eines Patienten und ein entsprechendes Scanprotokoll (14) zu empfangen;

wobei die Herzanatomie-Segmentierungseinheit dazu konfiguriert ist, die empfangenen koronaren Computertomographie-Angiographiedaten zu segmentieren, um eine Herzsegmentierungskarte (18) zu erzeugen, die mehrere anatomische Segmente einschließlich Myokardsegmenten darstellt;

wobei die Perfusionsdaten-Extraktionseinheit dazu konfiguriert ist, Perfusionsdaten (28) aus den empfangenen koronaren Computertomographie-Angiographiedaten zu extrahieren;

wobei die Vorhersageeinheit für koronare mikrovaskuläre Dysfunktion dazu konfiguriert ist, die empfangenen koronaren Computertomographie-Angiographiedaten, das entsprechende Scanprotokoll und patientenbezogene Daten einschließlich der erzeugten Herzsegmentierungskarte und der extrahierten Perfusionsdaten in ein statistisches Modell (34) einzugeben, um einen Indikator (36) für ein Vorhandensein einer koronaren mikrovaskulären Dysfunktion zu bestimmen;

wobei das statistische Modell ein Maschinenlernmodell ist, das dazu konfiguriert ist, eine Funktion zu verwenden, die die empfangenen koronaren Computertomographie-Angiographiedaten, das entsprechende Scanprotokoll und patientenbezogene Daten dem Indikator für ein Vorhandensein koronarer mikrovaskulärer Dysfunktion zuordnet, wobei das Maschinenlernmodell dazu konfiguriert ist, eine überwachte Lernmethodik zu verwenden, um die Funktion zu erstellen, die eine Eingabe basierend auf beispielhaften Eingabe-Ausgabe-Paaren einer Ausgabe zuordnet, wobei die beispielhaften Eingabe-Ausgabe-Paare Beispiele der koronaren Computertomographie-Angiographiedaten, das entsprechende Scanprotokoll und patientenbezogene Daten, einschließlich der erzeugten Herzsegmentierungskarte und der extrahierten Perfusionsdaten, als Eingaben und Beispiele des Indikators (36) als Ausgaben beinhalten; und

wobei die Ausgabeeinheit dazu konfiguriert ist, den Indikator für ein Vorhandensein koronarer mikrovaskulärer Dysfunktion auszugeben.

2. Entscheidungsunterstützungssystem nach Anspruch 1,
wobei das System dazu konfiguriert ist, den Indikator für ein Vorhandensein koronarer mikrovaskulärer Dysfunktion bei nicht-obstruktiven Koronargefäßen auszugeben, wenn das CCTA-Datenbild Koronargefäße eines Patienten ohne Obstruktionen enthält.

3. Entscheidungsunterstützungssystem nach Anspruch 1 oder 2,
wobei die koronaren Computertomographie-Angiographiedaten Folgendes umfassen:

   - konventionelle koronare Computertomographie-Angiographiedaten;
   - auf Photonenzählung basierende Computertomographie-Angiographiedaten; und/oder
   - spektrale koronare Computertomographie-Angiographiedaten mit mindestens zwei Energieniveaus.

4. Entscheidungsunterstützungssystem nach Anspruch 1 bis 3,
wobei die patientenbezogenen Daten ferner Folgendes umfassen:

   - eine Patientenkrankenakte; und/oder
   - klinische Daten.

5. Entscheidungsunterstützungssystem nach einem der vorstehenden Ansprüche,
wobei die Perfusionsdaten Folgendes umfassen:

   - eine Bewertung, die eine Wahrscheinlichkeit eines Vorhandenseins eines Perfusionsdefizits angibt; und/oder
   - eine Karte, die eine Wahrscheinlichkeit eines Perfusionsdefizits in jedem Myokardsegment der Herzsegmentierungskarte abbildet.

6. Entscheidungsunterstützungssystem nach einem der vorstehenden Ansprüche,
wobei die Perfusionsdaten-Extraktionseinheit dazu konfiguriert ist, ein Deep-Learning-Modell zu verwenden, um die Perfusionsdaten basierend auf den empfangenen koronaren Computertomographie-Angiographiedaten, dem entsprechenden Scanprotokoll und den patientenbezogenen Daten zu extrahieren.

7. Entscheidungsunterstützungssystem nach Anspruch 6,
wobei das Deep-Learning-Modell Folgendes umfasst:

   - ein Feedforward-Neuralnetz; und/oder
   - ein generatives kontradiktorisches Deep-Learning-Modell.

8. Entscheidungsunterstützungssystem nach Anspruch 7,
wobei das generative kontradiktorische Deep-Learning-Modell dazu konfiguriert ist, eine verbesserte Ruhe-Perfusionskarte zu erzeugen, sodass das Perfusionsdefizit, das auf einer Stress-Perfusionskarte sichtbar ist, auch auf der verbesserten Ruhe-Perfusionskarte sichtbar ist.

9. Entscheidungsunterstützungssystem nach Anspruch 7 oder 8,
wobei das generative kontradiktorische Deep-Learning-Modell dazu konfiguriert ist, unter Verwendung eines Prozesses für gleichzeitiges Training eines Generators zum Verbessern einer Ruhe-Perfusionskarte und eines Diskriminators zum Unterscheiden zwischen einem realen Stress-Perfusionsbild und einem verbesserten Ruhe-Perfusionsbild trainiert zu werden.

10. Entscheidungsunterstützungssystem nach einem der Ansprüche 6 bis 8,

   wobei das Deep-Learning-Modell dazu konfiguriert ist, unter Verwendung mindestens einer Referenz für ein Perfusionsdefizit trainiert zu werden; und
   wobei die mindestens eine Referenz für das Perfusionsdefizit Folgendes umfasst:

   - eine kardiale Magnetresonanz-Perfusionsmessung;
   - Stressperfusionsdaten;
   - Einzelphotonen-Emissionscomputertomographiedaten; und/oder

- Positronen-Emissionstomographiedaten.

11. Entscheidungsunterstützungssystem nach Anspruch 10, wobei das Maschinenlernmodell mindestens eines des Folgenden umfasst:

- ein tiefes neuronales Netzwerk;
- einen Regressionswald;
- einen Zufallswald; und
- eine Support-Vektor-Maschine.

12. Entscheidungsunterstützungssystem nach einem der vorstehenden Ansprüche, ferner umfassend:

- eine Anzeige, die dazu konfiguriert ist, den Indikator für ein Vorhandensein koronarer mikrovaskulärer Dysfunktion anzuzeigen.

13. Computerprogrammelement zum Steuern des Entscheidungsunterstützungssystems nach Anspruch 1, das, wenn es von einer Verarbeitungseinheit ausgeführt wird, dazu geeignet ist, ein Verfahren (100) zum Bereitstellen einer Beurteilung koronarer mikrovaskulärer Dysfunktion durchzuführen, das die folgenden Schritte umfasst:

- Bereitstellen (110) koronarer Computertomographie-Angiographiedaten eines Patienten und eines entsprechenden Scanprotokolls;
- Segmentieren (120) der empfangenen koronaren Computertomographie-Angiographiedaten, um eine Herzsegmentierungskarte zu erzeugen, die mehrere anatomische Segmente einschließlich Myokardsegmenten darstellt;
- Extrahieren (130) von Perfusionsdaten aus den empfangenen koronaren Computertomographie-Angiographiedaten; und
- Eingeben (140) der empfangenen koronaren Computertomographie-Angiographiedaten, des entsprechenden Scanprotokolls und patientenbezogener Daten einschließlich der erzeugten Herzsegmentierungskarte und der extrahierten Perfusionsdaten in ein statistisches Modell, um einen Indikator für ein Vorhandensein einer koronaren mikrovaskulären Dysfunktion zu bestimmen; und

wobei das statistische Modell ein Maschinenlernmodell ist, das dazu konfiguriert ist, eine Funktion zu verwenden, die die empfangenen koronaren Computertomographie-Angiographiedaten, das entsprechende Scanprotokoll und patientenbezogene Daten dem Indikator für ein Vorhandensein koronarer mikrovaskulärer Dysfunktion zuordnet, wobei das Maschinenlernmodell dazu konfiguriert ist, eine überwachte Lernmethodik zu verwenden, um die Funktion zu erstellen, die eine Eingabe basierend auf beispielhaften Eingabe-Ausgabe-Paaren einer Ausgabe zuordnet, wobei die beispielhaften Eingabe-Ausgabe-Paare Beispiele der koronaren Computertomographie-Angiographiedaten, das entsprechende Scanprotokoll und patientenbezogene Daten, einschließlich der erzeugten Herzsegmentierungskarte und der extrahierten Perfusionsdaten, als Eingaben und Beispiele des Indikators (36) als Ausgaben beinhalten.

## Revendications

1. Système d'aide à la décision (200) pour l'évaluation du dysfonctionnement microvasculaire coronarien, comprenant :

- une unité d'entrée (210) ;
- une unité de segmentation de l'anatomie cardiaque (220) ;
- une unité d'extraction de données de perfusion (230) ;
- une unité de prédiction du dysfonctionnement microvasculaire coronarien (240) ; et
- une unité de sortie (250) ;
dans lequel l'unité d'entrée est configurée pour recevoir les données d'angiographie par tomodensitométrie coronaire (12) d'un patient et un protocole de balayage correspondant (14) ;
dans lequel l'unité de segmentation de l'anatomie cardiaque est configurée pour segmenter les données d'angiographie par tomodensitométrie coronaire reçues afin de générer une carte de segmentation cardiaque (18) représentant plusieurs segments anatomiques, y compris des segments du myocarde ;
dans lequel l'unité d'extraction de données de perfusion est configurée pour extraire les données de perfusion (28) à partir des données d'angiographie par tomodensitométrie coronaire reçues ;

dans lequel l'unité de prédiction du dysfonctionnement microvasculaire coronarien est configurée pour entrer les données d'angiographie par tomodensitométrie coronarienne reçues, le protocole de balayage correspondant et les données relatives au patient, y compris la carte de segmentation cardiaque générée et les données de perfusion extraites, dans un modèle statistique (34) pour déterminer un indicateur (36) de la présence d'un dysfonctionnement microvasculaire coronarien ;

dans lequel le modèle statistique est un modèle d'apprentissage automatique configuré pour utiliser une fonction qui associe les données d'angiographie par tomodensitométrie coronaire reçues, le protocole d'acquisition correspondant et les données relatives au patient à l'indicateur de présence d'un dysfonctionnement microvasculaire coronaire, dans lequel le modèle d'apprentissage automatique est configuré pour utiliser une méthodologie d'apprentissage supervisé afin de créer la fonction qui associe une entrée à une sortie sur la base d'exemples de paires entrée-sortie, les exemples de paires entrée-sortie incluant des exemples de données d'angiographie par tomodensitométrie coronaire, de protocole d'acquisition correspondant et de données relatives au patient, y compris la carte de segmentation cardiaque générée et les données de perfusion extraites, en tant qu'entrées, et des exemples de l'indicateur (36), en tant que sorties ; et

dans lequel l'unité de sortie est configurée pour fournir l'indicateur de présence d'un dysfonctionnement microvasculaire coronarien.

2. Système d'aide à la décision selon la revendication 1,
dans lequel le système est configuré pour afficher l'indicateur de présence d'un dysfonctionnement microvasculaire coronaire pour les vaisseaux coronaires non obstructifs, lorsque l'image des données CCTA contient des vaisseaux coronaires d'un patient sans obstruction.

3. Système d'aide à la décision selon la revendication 1 ou 2,
dans lequel les données d'angiographie par tomodensitométrie coronaire comprennent :

- des données d'angiographie par tomodensitométrie coronarienne conventionnelle ;
- des données d'angiographie par tomodensitométrie basée sur le comptage de photons ; et/ou
- des données d'angiographie par tomodensitométrie coronarienne spectrale avec au moins deux niveaux d'énergie.

4. Système d'aide à la décision selon les revendications 1 à 3,
dans lequel les données relatives au patient comprennent en outre :

- un dossier médical du patient ; et/ou
- des données cliniques.

5. Système d'aide à la décision, conformément à l'une quelconque des revendications précédentes,
dans lequel les données de perfusion comprennent :

- un grade représentant une probabilité de présence d'un déficit de perfusion ; et/ou
- une carte représentant la probabilité d'un déficit de perfusion à chaque segment du myocarde de la carte de segmentation cardiaque.

6. Système d'aide à la décision, conformément à l'une quelconque des revendications précédentes,
dans lequel l'unité d'extraction des données de perfusion est configurée pour utiliser un modèle d'apprentissage profond afin d'extraire les données de perfusion à partir des données d'angiographie par tomodensitométrie coronaire reçues, du protocole de balayage correspondant et des données relatives au patient.

7. Système d'aide à la décision selon la revendication 6,
dans lequel le modèle d'apprentissage profond comprend :

- un réseau neuronal à propagation directe ; et/ou
- un modèle d'apprentissage profond génératif adverse.

8. Système d'aide à la décision selon la revendication 7,
dans lequel le modèle d'apprentissage profond génératif antagoniste est configuré pour générer une carte de perfusion au repos améliorée afin que le déficit de perfusion, visible sur une carte de perfusion sous stress, soit visible sur la carte de perfusion au repos améliorée.

**9.** Système d'aide à la décision selon la revendication 7 ou 8,
dans lequel le modèle d'apprentissage profond génératif adverse est configuré pour être entraîné à l'aide d'un processus permettant d'entraîner simultanément un générateur pour améliorer une carte de perfusion au repos et un discriminateur pour distinguer entre une image de perfusion sous stress réelle et une image de perfusion au repos améliorée.

**10.** Système d'aide à la décision selon l'une quelconque des revendications 6 à 8,

dans lequel le modèle d'apprentissage profond est configuré pour être entraîné à l'aide d'au moins une référence pour un déficit de perfusion ; et
dans lequel l'au moins une référence relative au déficit de perfusion comprend :

- une mesure de perfusion par résonance magnétique cardiaque ;
- des données de perfusion sous stress ;
- des données de tomographie d'émission monophotonique ; et/ou
- des données de tomographie par émission de positons.

**11.** Système d'aide à la décision selon la revendication 10,
dans lequel le modèle d'apprentissage automatique comprend au moins un des éléments suivants :

- un réseau neuronal profond ;
- une forêt de régression ;
- une forêt aléatoire ; et
- une machine à vecteurs de support.

**12.** Système d'aide à la décision selon l'une quelconque des revendications précédentes, comprenant en outre :

- un écran configuré pour afficher l'indicateur de présence d'un dysfonctionnement microvasculaire coronarien.

**13.** Élément de programme informatique destiné à commander le système d'aide à la décision selon la revendication 1, qui, lorsqu'il est exécuté par une unité de traitement, est adapté pour réaliser un procédé (100) d'évaluation du dysfonctionnement microvasculaire coronarien, comprenant les étapes suivantes :

- la fourniture (110) de données d'angiographie par tomodensitométrie coronaire d'un patient et d'un protocole de balayage correspondant ;
- la segmentation (120) des données d'angiographie par tomodensitométrie coronaire reçues pour générer une carte de segmentation cardiaque représentant plusieurs segments anatomiques, y compris des segments du myocarde ;
- l'extraction (130) des données de perfusion à partir des données d'angiographie par tomodensitométrie coronaire reçues ; et
- la saisie (140) des données d'angiographie par tomodensitométrie coronaire reçues, du protocole d'acquisition correspondant et des données relatives au patient, y compris la carte de segmentation cardiaque générée et les données de perfusion extraites, dans un modèle statistique afin de déterminer un indicateur de présence d'un dysfonctionnement microvasculaire coronaire ; et

dans lequel le modèle statistique est un modèle d'apprentissage automatique configuré pour utiliser une fonction qui mappe les données d'angiographie par tomodensitométrie coronaire reçues, le protocole de balayage correspondant et les données relatives au patient à l'indicateur de présence d'un dysfonctionnement microvasculaire coronaire, dans lequel le modèle d'apprentissage automatique est configuré pour utiliser une méthodologie d'apprentissage supervisé afin de créer la fonction qui mappe une entrée à une sortie sur la base d'exemples de paires entrée-sortie, les exemples de paires entrée-sortie incluant des exemples de données d'angiographie par tomodensitométrie coronaire, du protocole de balayage correspondant et des données relatives au patient, y compris la carte de segmentation cardiaque générée et les données de perfusion extraites, comme entrées, et des exemples de l'indicateur (36), comme sorties.

100

110

120

130

140

# FIG. 1

12
Input data (1)
• (Spectral) CCTA scan

14
Input data (2)
• Scan protocol

• Patient medical records
• Clinical data

16

10
Deep neural net:

18
Output:
• Segmen-tation map

# FIG. 2A

FIG. 2B

FIG. 2C

**FIG. 3A**

**FIG. 3B**

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017046082 A1
- US 2018276817 A1

- US 9349178 B1